# NEUE EUROPÄISCHE PATENTSCHRIFT

(11) **EP 0 691 123 B2**
(45) Veröffentlichungstag und Bekanntmachung des Hinweises auf die Entscheidung über den Einspruch: **26.09.2001**
(45) Hinweis auf die Patenterteilung: 19.11.1997
(21) Anmeldenummer: 94810394.0
(22) Anmeldetag: 05.07.1994
(51) Int. Cl.: A61K 6/04, C22C 5/02

(54) **Hochgoldhaltige Dentallegierung**
Dental alloy with a high gold content
Alliage dentaire à haute teneur en or

(43) Veröffentlichungstag der Anmeldung: 10.01.1996
(73) Patentinhaber: CENDRES ET METAUX S.A., CH-2501 Bienne (CH)
(72) Erfinder: Fischer, Jens, Dr. Dr., CH-3038 Kirchlindach (CH)
(74) Vertreter: AMMANN PATENTANWAELTE AG BERN

(56) Entgegenhaltungen:
- DE-A- 2 302 837
- DE-A- 2 357 552
- DE-A- 2 424 575
- DE-B- 2 828 304
- DE-C- 4 419 408
- NL-A- 9 200 564
- US-A- 4 606 981
- Informed Consent, Quarterly Publication of the Environmental Dental Association, Winter 1992, Issue 2, S.5
- Rundschreiben der Firma Pure Alloy Laboratories, Inc. September 1993
- Metalle in der Zahntechnik, Verlag Neuer Merkur GmbH, München, 16. Auflage, 1992, S.66-67
- Lexikon der Zahntechnik, Verlag Neuer Merkur GmbH, München, 4. Auflage, 1978, S.221

## Beschreibung

Die vorliegende Erfindung betrifft die Verwendung einer hochgoldhaltigen Dentallegierung von goldgelber Farbe für die Verblendung mit handelsüblichen Dentalkeramikmassen und zur Herstellung von unverblendeten oder mit Kunststoff oder anderen Materialien zu verblendenden Zahnprotheseteilen.

Hochgoldhaltige Edelmetall-Dentallegierungen sind für den Einsatz bei metallischem, festsitzendem Zahnersatz wie Kronen, Brücken usw. unter anderem wegen ihrer guten biologischen Verträglichkeit und ihrer hohen Korrosionsresistenz im Mundmilieu weit verbreitet. Überdies lassen sie sich technisch gut verarbeiten.

Silber- und kupferhaltige Goldgusslegierungen werden bereits seit langem erfolgreich in der restaurativen Zahnheilkunde eingesetzt. Die erforderliche hohe mechanische Festigkeit dieser Materialien wird bei den konventionellen Legierungen für unverblendete oder mit Kunststoff zu verblendenden Arbeiten über das Silber-Kupfer-Verhältnis eingestellt.

Im Hinblick auf eine optimale Aesthetik festsitzender dentaler Rekonstruktionen, insbesondere im sichtbaren Bereich, hat sich die mindestens teilweise Verblendung einer metallischen Basis mit keramischer Masse als sehr gut geeignet herausgestellt, da in diesem Werkstoffverbund die Vorteile der Keramik, nämlich Härte, Aesthetik und hervorragende Biokompatibilität, mit den Vorteilen des metallischen Werkstoffes, nämlich Zugfestigkeit und bessere Passgenauigkeit, optimal kombiniert werden können.

Die Verwendung einer keramischen Verblendung erfordert von der Legierung spezielle Eigenschaften. So muss das Schmelzintervall der Legierung deutlich über der Brenntemperatur der Keramik liegen, die ca. 980 °C beträgt, und die Legierung muss darüberhinaus eine ausreichende Brennstabilität aufweisen, damit die für die Verblendung dienende metallische Basis während des Brennprozesses formtreu bleibt.

Damit ein dauerhafter Verbund zwischen Legierung und Keramik gewährleistet ist, sollte in der Keramik während des Herstellungsverfahrens keine Zugspannung aufgebaut werden. Dies wird bekanntermassen dadurch erreicht, dass der Wärmeausdehnungskoeffizient der Legierung leicht über dem Wärmeausdehnungskoeffizienten der Keramik liegt. Während des Abkühlungsprozesses wird die Keramik dann durch die etwas stärker schrumpfende Legierung unter Druckspannung versetzt.

Die genannten Anforderungen führten zur Entwicklung von speziellen, sogenannten Aufbrennlegierungen, die heute neben den konventionellen Goldgusslegierungen eine eigene Klasse bilden und über die Normen ISO 9693 und DIN 13927 genormt sind.

Um die erforderlichen, oben genannten Eigenschaften von Aufbrennlegierungen zu erreichen, hat man den Legierungen für die Metallkeramik auf der Basis von Gold die Elemente Platin und/oder Palladium zulegiert. Darüber hinaus werden zur erforderlichen Festigkeitssteigerung der Legierung Nichtedelmetalle zulegiert, insbesondere Kupfer, Indium, Gallium, Zinn und/oder Zink. Eine an sich mögliche Festigkeitssteigerung über grössere Anteile an Silber und Kupfer ist wegen unerwünschter Reaktionen solcher Legierungen mit der Keramik und einer zu starken Oxidation der Legierung nicht anwendbar.

Insbesondere Palladium- aber auch Platinzusätze führen zu einer deutlichen Reduktion der erwünschten gelben Farbe der Legierung, welche vom Patienten im allgemeinen als ästhetisch ansprechend und erstrebenswert empfunden wird.

In jüngster Zeit sind einige der in den Edelmetall-Legierungen verwendeten Nichtedelmetalle in den Verdacht geraten, bei einigen Patienten Beschwerden und pathologische Reaktionen verursacht zu haben. Insbesondere sei hier auf das Indium verwiesen, das in fast allen aufbrennfähigen Legierungen enthalten ist, Literatur: Wirz, J.: Schädigung des Parodontes durch zahnärztliche Werkstoffe. Zahnärztliche Welt 102, 146 (1993). Auch Palladium ist in den Verdacht geraten, toxische oder allergische Reaktionen hervorzurufen, wenn es in höheren Konzentrationen in der Edelmetall-Legierung enthalten ist und deshalb durch Korrosion freigesetzt werden kann. Daneben kommt zunehmend der Wunsch nach einer universell einsetzbaren Dentallegierung auf, die sowohl für konventionelle, unverblendete bzw. mit Kunststoff verblendete Zahnprothesen, als auch für die Technik der Metallkeramik geeignet ist. Solche Legierungen haben den Vorteil, dass die Gefahr der Bildung eines galvanischen Elementes infolge der Verwendung verschiedener Legierungen in der Mundhöhle mit den damit verbundenen Korrosionsvorgängen ausgeschlossen ist. Neuerdings werden derartige Universallegierungen angeboten, die jedoch auf der Basis Au-Ag-Pt-Cu mit Zusätzen der Nichtedelmetalle Indium und Zink entwickelt wurden. Diese Legierungen haben den Nachteil, dass sie aufgrund des hohen Nichtedelmetall-Gehaltes relativ korrosionsanfällig sind, insbesondere durch das beim Brand entstehende Oberflächenoxid, welches im Bereich des Kronenrands nicht unbedingt vollständig von Keramik bedeckt ist und deshalb dem Speichel und seiner korrosiven Wirkung zugänglich ist. Weiterhin wird für diese Legierung eine spezielle niedrigschmelzende Keramik benötigt, die abhängig vom Herstellungsverfahren eine höhere Korrosionsrate aufweist als die bekannten höherschmelzenden Verblendkeramiken.

Darüberhinaus zeigen hochgoldhaltige Legierungen generell eine schlechte Hochtemperatur-Kriechfestigkeit, so dass sich in der Regel metallische Brückengerüste mit langen Spannweiten während des Brennprozesses verformen und ihre Passgenauigkeit verlieren. Deshalb ist man für lange, mit Keramik zu verblendene Brücken auf Legierungen mit einem höheren Palladiumgehalt angewiesen, die allerdings nicht mehr. die gewünschte und von den Patienten geschätzte goldgelbe Farbe haben und die genannten biologischen Nachteile aufweisen.

Auf der anderen Seite haben sich die Nichtedelmetalle Titan und Tantal als äusserst biokompatible Materialien in der Zahnheilkunde bewährt. Implantate aus Titan beispielsweise heilen im Knochen aufgrund der oberflächlichen Bildung des sehr korrosionsstabilen und inerten Titanoxids problemlos ohne jegliche Abwehrreaktion ein und allergische Reaktionen auf dieses Material kommen - wenn überhaupt - nur extrem selten vor. Daher sind diese Nichtedelmetalle aus klinischbiologischer Sicht als ideale Legierungspartner für das bekanntermassen ebenfalls extrem korrosionsstabile Gold anzusehen.

Aus der Patentliteratur sind bereits hochgoldhaltige Dentallegierungen mit Titan bekannt, die sich zum Verblenden mit Keramik eignen. Die DE-A-2 302 837 beschreibt eine hochgoldhaltige Titanlegierung, die ausserdem einen relativ hohen Platin- oder Platingruppenanteil sowie Palladium aufweist, bei letzterem vor allem Palladium, das wie weiter oben beschrieben allergische Reaktionen hervorrufen kann. Ausserdem beschreibt die unter DE-A-2 357 552 veröffentlichte Nachfolgeanmeldung eine hochgoldhaltige Titanlegierung, die ebenfalls ein Element aus der Platingruppe aufweist, wobei keine mengenmässige Angabe dazu gemacht wird.

Ausserdem sind hochgoldhaltige Titanlegierungen für die Schmuckindustrie bekannt, beispielsweise aus der EP-A-190 648. Bei diesen Schmucklegierungen geht es jedoch um andere Kriterien als bei Dentallegierungen und dafür offenbarte der Zusammensetzungen können nicht ohne weiteres, siehe weiter oben, auf Dentallegierungen übertragen werden.

Dies trifft auch auf die duktilen Lötfolien auf der Basis einer Titan-Goldlegierung gemäss US-A-4 606 981 zu, in welcher Patentschrift nur diese für Lötzwecke geeigneten duktilen Eigenschaften offenbart und diskutiert.

Es ist von diesem Stand der Technik ausgehend Aufgabe der vorliegenden Erfindung eine Dentallegierung anzugeben, die nicht nur eine hervorragende Biokompatibilität aufweist sondern auch universell einsetzbar ist und den Anforderungen der Normen ISO 1562 und ISO 9693 bzw. DIN 13927 gerecht wird. Diese Aufgabe wird mit einer Legierung gelöst, die in Patentanspruch 1 definiert ist.

Versuche haben ergeben, dass eine Legierung, die sich aus 91 - 99,4 % Gold, 0,5 - 3 % Tantal, 0- 5 % Silber, 0 - 1 % Iridium, Rhodium, Ruthenium, Platin, Palladium, Osmium, Wolfram, Eisen, Molybdän, Niob und/oder Rhenium zusammensetzt, überraschend universelle Eigenschaften aufweist und daher auch in der konventionellen Technik angewendet werden kann und die den Anforderungen der Normen ISO 1562 und ISO 9693 bzw. DIN 13927 gerecht werden. Da sowohl Gold als auch Tantal äusserst biokompatibel sind, ist auch eine Legierung dieser beiden Komponenten extrem biokompatibel und ausserdem ästhetisch sehr ansprechend, da das Tantal in den genannten Mengen die Goldfarbe nicht wesentlich beeinflussen. Ausserdem enthält diese Legierung einen sehr kleinen Anteil von Nichtedelmetallen, wodurch sie ihre Korrosionsstabilität nicht verliert.

Ueberraschend hat sich zudem gezeigt, dass Legierungen der genannten Zusammensetzung eine äusserst gute Hochtemperatur-Kriechfestigkeit zeigen. So hat z. B. eine Legierung mit der Zusammensetzung 97,5 - 98,5 % Gold, 1,4 - 2,4 % Titan, 0,05 - 0,15 % Iridium eine Hochtemperatur-Kriechfestigkeit, die besser ist als diejenige aller bisher im Dentalbereich angewendeten Edelmetall-Gusslegierungen, so dass mit dieser Legierung erstmals im Gussverfahren hergestellte lange metallische Brückgerüste aus einer hochgoldhaltigen, goldgelben Legierung problemlos mit Keramik verblendet werden können. Es hat sich gezeigt, dass durch das Zulegieren von mehr als 1 % Platin, wie es beispielsweise in der DE-A-2 302 837 beschrieben ist, die Hochtemperatur-Kriechfestigkeit deutlich reduziert wird.

## Patentansprüche

1. Verwendung einer hochgoldhaltigen Dentallegierung zur Verblendung mit Dentalkeramikmassen und zur Herstellung von Zahnprothesenteilen, wobei die Dentallegierung aus 91 - 99,4 % Gold, 0,5 - 3 % Tantal, 0 - 5 % Silber, 0 - 1 % Iridium, Rhodium, Ruthenium, Platin, Palladium, Osmium, Wolfram, Eisen, Molybdän, Niob, und/oder Rhenium besteht und die Prozentangaben Gewichtsprozente sind.

2. Hochgoldhaltige Dentallegierung, **dadurch gekennzeichnet, dass** sie aus 97,5 - 98,5 % Gold, 1,4 - 2,4 % Titan und 0,05 - 0,15 % Iridium besteht.

## Claims

1. Use of a dental alloy with a high gold content for blending with ceramic dental compositions and for the preparation of parts of dental replacements, the dental alloy being composed of 91 to 99.4% of gold, of 0.5 to 3% of tantalum, of 0 to 5% of silver, of 0 to 1% of iridium, rhodium, ruthenium, platinum, palladium, osmium, tungsten, iron, molybdenum, niobium, and/or rhenium, and the percentages being weight percentages.

2. Dental alloy with a high gold content, **characterised in that** it is composed of 97.5 to 98.5% of gold, of 1.4 to 2.4% of titanium, and of 0.05 to 0.15% of iridium.

## Revendications

1. Utilisation d'un alliage dentaire à haute teneur en or pour le mélange intime à des compositions céramiques dentaires et pour la préparation d'éléments de prothèses dentaires, l'alliage dentaire étant composé de 91 à 99,4% d'or, de 0,5 à 3% de tantale, de 0 à 5% d'argent, de 0 à 1% d'iridium, de rhodium, de ruthénium, de platine, de palladium, d'osmium, de tungstène, de fer, de molybdène, de niobium, et/ou de rhénium, et les pourcentages étant des pourcentages en poids.

2. Alliage dentaire à haute teneur en or, **caractérisé en ce qu'**il est composé de 97,5 à 98,5% d'or, de 1,4 à 2,4% de titane et de 0,05 à 0,15% d'iridium.
